# EUROPEAN PATENT APPLICATION

(11) **EP 4 227 419 A1**
(43) Date of publication of application: **16.08.2023**
(21) Application number: 23155899.0
(22) Date of filing: 09.02.2023
(51) Int. Cl.: C12P 19/14, C12P 19/16, C12M 1/09, C12M 1/00

(54) **METHOD FOR SACCHARIFYING CELLULOSE**

(30) Priority: 10.02.2022 JP 2022019356
(71) Applicant: Seiko Epson Corporation, Tokyo 160-8801 (JP)
(72) Inventor: YAMASAKI, Sayaka, Suwa-shi, 392-8502 (JP); TANAKA, Hideki, Suwa-shi, 392-8502 (JP); NAKAI, Yoko, Suwa-shi, 392-8502 (JP); NAKASHIMA, Yoshiki, Suwa-shi, 392-8502 (JP)
(74) Representative: Lewis Silkin LLP

(57) **Abstract**

A method for saccharifying cellulose includes introducing a cellulose-containing raw material and a reaction solution into a saccharification tank, introducing an enzyme into the reaction solution in the saccharification tank, disposing an adsorption sheet on the liquid surface of the reaction solution, and decomposing the cellulose with the enzyme by stirring the reaction solution to produce a saccharide. The adsorption sheet adsorbs an oil component released from the raw material to the liquid surface of the reaction solution during the production of the saccharide.

## Description

The present application is based on, and claims priority from JP Application Serial Number 2022-019356, filed February 10, 2022, the disclosure of which is hereby incorporated by reference herein in its entirety.

### BACKGROUND

### 1. Technical Field

The present disclosure relates to a method for saccharifying cellulose.

### 2. Related Art

From the viewpoint of so-called carbon minus, effective utilization of biomass, application thereof to bioethanol, etc., attention is paid to production of glucose by saccharification of cellulose. For example, JP-T-2014-507148 discloses a method for producing a saccharide by saccharification using paper as a raw material. The technique described in JP-T-2014-507148 discloses a method for producing a saccharide using used paper as a cellulose raw material through pre-treatment and saccharification with an enzyme.

In order to saccharify cellulose, there is a method for saccharifying cellulose, in which a saccharification reaction solution, containing an enzyme, and a raw material are added into a saccharification reaction tank, and enzyme reaction is allowed to proceed by stirring. However, when paper is used as a cellulose raw material, the paper contains not only cellulose but also pitch (adhesive material), and when the paper is used paper, the paper contains components insoluble in the saccharification reaction solution, such as an ink oil component. These components are not saccharified, and thus not only may inhibit saccharification reaction due to the adhesion to cellulose and inhibition of the enzyme reaction, but also may remain in the reaction solution and thus remain as an unnecessary component, such as sludge or the like, on the inner wall of the saccharification tank.

Therefore, there is required a method for saccharifying cellulose, in which the enzyme reaction is hardly inhibited by impurities such as oil components, including lignin, pitch, and the like, in the raw material, and the inside of the saccharification tank is easily maintained clean.

### SUMMARY

According to an aspect of the present disclosure, a method for saccharifying cellulose includes introducing a cellulose-containing raw material and a reaction solution into a saccharification tank, introducing an enzyme into the reaction solution in the saccharification tank, disposing an adsorption sheet on the liquid surface of the reaction solution, and decomposing the cellulose with the enzyme by stirring the reaction solution to produce a saccharide, the adsorption sheet adsorbing oil components released from the raw material to the liquid surface of the reaction solution during the production of the saccharide.

### BRIEF DESCRIPTION OF THE DRAWINGS

FIG. 1 is a schematic view showing the outlines of saccharification treatment according to an example.
FIG. 2 is a schematic plan view of an adsorption sheet according to an example.
FIG. 3 is a schematic plan view of an adsorption sheet according to a modified example.

### DESCRIPTION OF EXEMPLARY EMBODIMENTS

An exemplary embodiment of the present disclosure is described below. In the description of the exemplary embodiment below, an example of the present disclosure is described. The present disclosure is not limited to the exemplary embodiment below and includes various modified embodiments carried out within a range which does not change the gist of the present disclosure. In addition, all configurations described below are not necessarily configurations essential for the present disclosure.

A method for saccharifying cellulose according to an exemplary embodiment of the present disclosure includes introducing a cellulose-containing raw material and a reaction solution into a saccharification tank, introducing an enzyme into the reaction solution in the saccharification tank, disposing an adsorption sheet on the liquid surface of the reaction solution, and producing a saccharide in the reaction solution by stirring the reaction solution.

### 1. Introduction of raw material and reaction solution into saccharification tank

The cellulose-containing raw material and the reaction solution are introduced into the saccharification tank.

### 1. 1. Saccharification tank

The saccharification tank is not particularly limited as long as the raw material and the reaction solution can be introduced, and the contents can be stirred. Also, the scale of the saccharification tank is not limited, and it may be a laboratory scale such as a beaker, a flask, or the like, or may be a pilot plant scale or a commercial plant scale.

The saccharification tank may include a container and a lid body. The saccharification tank may properly include an inlet for the raw material and the reaction solution, an outlet for products, a mechanism for internal stirring, a window for internal observation, a heater for heating/cooling, and other piping such as refrigerant piping, a jacket, and the like. Further, the saccharification tank may include a liquid level meter, a thermometer, and the like and may include openings for installing them.

Examples of the stirring mechanism include a magnetic stirrer, a stir bar, a motor for stirring, a shaft, a stirring blade, and the like, and the stirring mechanism can be properly selected according to the scale and the stirring efficiency of contents.

### 1. 2. Raw material

The raw material introduced into the saccharification tank contains cellulose. The raw material may contain a component other than cellulose. Examples of the component other than cellulose include wood-derived components, such as lignin and hemicellulose, processed-wood components, such as a filler, a pigment, a resin component, clays, a binder, a toner, oil, moisture, and the like.

The raw material may be paper, pulp, or the like. When paper is used as the raw material, the raw material can be easily procured. Also, paper may contain printed used paper. Examples of the printed used paper include copy paper, newspaper, a magazine, and the like. The use of printed used paper is preferred because wastes can be decreased due to saving of environmental resources and underground resources or the like.

The raw material may be introduced in a crushed state into the saccharification tank. Crushing may be performed by, for example, cutting with a shredder or the like, or grinding with a dry defibering machine or the like. Further, crushing may be performed by, for example, wet disaggregation. When the raw material is in a crushed state, the components other than cellulose contained in the raw material are easily released from the cellulose and the cellulose easily comes into contact with the reaction solution, and thus the efficiency of saccharification can be improved.

The raw material is more preferably used in a sterilized state. Examples of a sterilization method include heating, ultraviolet irradiation, and the like. This makes it difficult that the glucose or the like produced by saccharification reaction is consumed by microorganisms or the like derived from the raw material, and thus the yield of saccharide may be increased.

### 1. 3. Reaction solution

The reaction solution contains water as a main component. Besides the water, the reaction solution may contain, for example, a material useful for enzyme reaction, a pH adjuster, a surfactant, etc.

The water is preferably pure water or ultrapure water from which ionic impurities are moved as much as possible, such as ion exchange water, ultrafiltered water, reverse osmosis water, distilled water, or the like. Also, water sterilized by ultraviolet irradiation, hydrogen peroxide addition, or the like is preferably used because when the reaction solution is stored for a long period time, the occurrence of mold or bacteria can be suppressed during enzyme reaction or after enzyme reaction.

Examples of the pH adjuster include organic acids such as acetic acid, citric acid, phosphoric acid, and the like; inorganic acids, organic alkali, inorganic alkali, and salts thereof such as sodium salt, one or more of which are selected, a material which constitutes a buffer solution, and the like.

The surfactant can be used without being particularly limited as long as it does not inhibit the enzyme reaction. Examples of the surfactant include Triton X-100 (manufactured by Nacalai Tesque, Inc), Antifoam SE-15 (manufactured by Sigma Aldrich Japan G. K.), Adekanol LG-126 (manufactured by ADEKA Corporation), DK Ester F-10 (manufactured by DKS Co., Ltd.), KM-72 (manufactured by Shin-Etsu Chemical Co., Ltd.), and the like. When the saccharification raw material such as dry used paper is mixed with the saccharification reaction solution, saccharification reaction may be inhibited by adhesion of air bubbles to the surface of the saccharification raw material. However, inhibition of the saccharification reaction may be suppressed by adding the surfactant.

### 1. 4. Mode of introduction

Also, a method for introducing the raw material and the reaction solution into the saccharification tank is not particularly limited. With respect to the order of introduction into the saccharification tank, either the raw material or the reaction solution may be first introduced or both may be simultaneously introduced.

### 2. Introduction of enzyme into reaction solution

An enzyme is introduced into the reaction solution at least before saccharification reaction.

### 2. 1. Enzyme

Any enzyme can be used as long as it has the function of decomposing cellulose into a saccharide by cutting a β-1,4-glucosidic linkage. Examples of a cellulose-decomposing enzyme include endoglucanase, cellobiohydrolase, cellobiase (β-glucosidase), and the like. More specific examples of the cellulose-decomposing enzyme include cellulase SS (manufactured by Nagase Chemtex Corporation), Accellerase Duet (manufactured by GENENCOR Inc.), Cellic Ctec 2 (manufactured by Novozymes Ltd.), Cellic Ctec 3 (manufactured by Novozymes Ltd.), Meicelase (manufactured by Meiji Seika Pharma Co., Ltd.), and the like. These enzymes may be used in combination of plural types. The enzyme may contain xylanase for enhancing the efficiency of saccharification by simultaneously decomposing xylane present on the surface of cellulose.

### 2. 2. Mode of introduction

The enzyme may be introduced as a solution or a dispersion liquid into the reaction solution or introduced as a powder. The enzyme may be introduced after the raw material and the reaction solution are introduced into the saccharification tank or may be mixed in advance with the reaction solution or the raw material and then introduced together with the reaction solution and the raw material.

### 3. Disposition of adsorption sheet on liquid surface in saccharification tank

The adsorption sheet is disposed on the liquid surface in the saccharification tank in which at least the raw material and the reaction solution have been introduced. The adsorption sheet may be fixed to the saccharification tank or may be moved in the saccharification tank by stirring described later. Also, the adsorption sheet may be disposed on the liquid surface by the buoyancy of the reaction solution in the saccharification tank or disposed on the liquid surface by using a structural material or the like. 3. 1. Adsorption sheet

The adsorption sheet adsorbs impurities such as oil components, including lignin, pitch, and the like, in the raw material. The adsorption sheet may adsorb impurities such as not only oil components, including lignin, pitch, and the like, in the raw material, but also oil components released from the raw material by enzyme reaction. The adsorption sheet can physically adsorb and collect the impurities such as oil components and the like. The adsorption sheet need not cover the entire liquid surface in the saccharification tank and may be disposed on at least a portion of the liquid surface. In addition, the number of adsorption sheets disposed on the liquid surface may be one or plural.

### Material of adsorption sheet

The material of the adsorption sheet is not particularly limited as long as it does not deteriorate during the enzyme reaction and has affinity for oil components. That is, the material of the adsorption sheet may be one which hardly causes corrosion at pH close to optimum pH for the enzyme used in enzyme reaction and which can adsorb or absorb the oil components on the surface or inside. Examples of such a material include materials which are hardly corroded at pH close to optimum pH for the enzyme, such as a metal, carbon, an inorganic material, resins, and a composite material thereof. More specific examples thereof include styrene, polyethylene, polypropylene, polytetrafluoroethylene, polyurethane, polyester, polybutadiene, styrene-butadiene rubber, silicone rubber, natural rubber, carbon fibers, activated carbon, and the like, and a composite material of a plurality of these materials. Structure of adsorption sheet

The adsorption sheet adsorbs the oil components and preferably has a larger surface area because the efficiency of adsorption is increased with increases in the surface area. From the viewpoint of this, the adsorption sheet preferably has a structure having a large specific surface area, for example, a network structure, a woven fabric structure, a non-woven fabric structure, a sponge structure, a porous structure, or the like. When the adsorption sheet has a network structure, the mesh size of network may be, for example, 0.1 mm or more and 10 mm or less, preferably 0.5 mm or more and 5 mm or less, and more preferably 1 mm or more and 3 mm or less. This enables to obtain the liquid permeability of the adsorption sheet while improving adsorption efficiency. Thus, for example, even when the entire portion of the liquid surface is covered with the adsorption sheet, for example, an enzyme solution can be introduced or added to the reaction solution through meshes.

### Macroscopic shape of adsorption sheet

Also, the overall shape of the adsorption sheet is not particularly limited. The thickness of the adsorption sheet is, for example, 10 µm or more and 5 cm or less, preferably 100 µm or more and 3 cm or less, and more preferably 1 mm or more and 1 cm or less. The thickness of the adsorption sheet can be set to such a degree that the shape or stiffness can be maintained according to the material and structure of the adsorption sheet. This can facilitate the handling of the adsorption sheet.

The shape in a plan view of the adsorption sheet is not particularly limited. The adsorption sheet may have a hole in a plan view or have a shape having a notch or cut. The shape in a plan view of the adsorption sheet can be properly designed according to conditions such as the shape of the saccharification tank, the supply and addition of the raw material and the enzyme, the configuration of the stirring mechanism, handling of the adsorption sheet, washing of the saccharification tank, etc.

When the saccharification tank has a cylindrical shape and the liquid surface in the saccharification tank has a circular shape in a plan view, the adsorption sheet preferably has an annular shape in a plan view. Therefore, through the central hole of the annulus of the adsorption sheet, the shaft of the stirring blade can be passed, and an additional component can be introduced into the saccharification tank, and thus the saccharification efficiency can be further enhanced.

### 3. 2. Variation of disposition

The adsorption sheet is more preferably disposed near the inner wall of the saccharification tank. Thus, the adsorption sheet can be disposed near the inner wall of the saccharification tank where impurities such as oil components, including lignin, pitch, and the like, in the raw material are easily gathered by stirring. This can more increase the efficiency of collection of impurities by the adsorption sheet.

### 4. Production of saccharide

The cellulose is decomposed with the enzyme by stirring the reaction solution to produce a cellulose-derived saccharide. The production is performed for a time of 2 hours or more and within 1 week depending on the ability of the enzyme and the overall scale. Typically, the production is performed for a time of 10 hours or more and within 5 days and more preferably 1 day or more and within 4 days.

### 4. 1. Stirring

Stirring is performed by the proper stirring mechanism described above. During stirring, the rotational speed or the like of the motor can be property set according to the scale and configuration of the saccharification tank, the shape of the stir bar or stirring blade, etc.

### 4. 2. pH

The pH of the reaction solution is adjusted according to pH optimum for the enzyme introduced. For example, when the enzyme used is Cellic Ctec 2 (manufactured by Novozymes Ltd.), the pH of the liquid in the saccharification tank is 4.5 or more and 6 or less and preferably 5.0 or more and 5.7 or less. The pH is more preferably adjusted before the enzyme is introduced in the saccharification tank.

The pH can be adjusted by adding sodium acetate, acetic acid, sulfuric acid, an aqueous sodium hydroxide solution or the like to the reaction solution. Also, in a configuration in which the pH of the reaction solution can be monitored, the pH may be adjusted during stirring. For example, when the pH becomes higher than the predetermined pH during stirring, the pH can be adjusted by adding acetic acid, sulfuric acid, or the like, while when the pH becomes lower than the predetermined pH, the pH can be adjusted by adding an aqueous sodium hydroxide solution or the like.

In addition, when the adsorption sheet has a hole, an acid, an alkali, or the like for adjusting the pH can be easily added to the liquid through the hole.

### 4. 3. Temperature

In producing a saccharide, the temperature of the liquid in the saccharification tank is preferably adjusted to a temperature optimum for the enzyme used. For example, when the enzyme used is Cellic Ctec 2 (manufactured by Novozymes Ltd.), the temperature of the liquid in the saccharification tank is 47°C or more and 52°C or less, preferably 48°C or more and 51°C or less, and more preferably 49°C or more and 50°C or less.

The temperature of the liquid in the saccharification tank is adjusted by using a suitable heating device, a cooling device, a control device, or the like.

### 5. Other operations

The saccharification method according to the exemplary embodiment may include pretreatment, recovery, washing, etc. other than the above.

### 5. 1. Pretreatment

The saccharification method may include pretreatment. Examples of the pretreatment include sterilization of the raw material and the reaction solution, grinding of the raw material, classification of the raw material, and the like.

### 5. 2. Recovery

The saccharification method may include recovering the liquid in the saccharification tank after the saccharide is produced. The recovery may be performed through piping or the like. The recovered liquid containing the saccharide may be filtered. Also, the used adsorption sheet may be recovered at the time of recovery.

### 5. 3. Washing of saccharification tank

The saccharification method may include washing the saccharification tank after the saccharide is produced. The washing of the saccharification tank may include, before and/after recovery, removing the precipitates remaining in the saccharification tank, washing the oil components remaining in the saccharification tank, washing the stirring mechanism, and the like.

### 6. Operation and effect

The saccharification method according to the exemplary embodiment enables the adsorption sheet to collect impurities such as oil components, including lignin, pitch, and the like, in the raw material, and thus the enzyme reaction is hardly inhibited, and the inside of the saccharification tank can be easily maintained clean. Therefore, cellulose can be efficiency saccharified, and thus a saccharide can be efficiency produced. Also, the inside of the saccharification tank is cleaner, and thus the saccharification tank after use can be easily washed. 7. Experimental example, etc.

The present disclosure is described in further detail below by an experimental example etc., but the present disclosure is not limited to these examples.

### 7. 1. Examples of saccharification tank

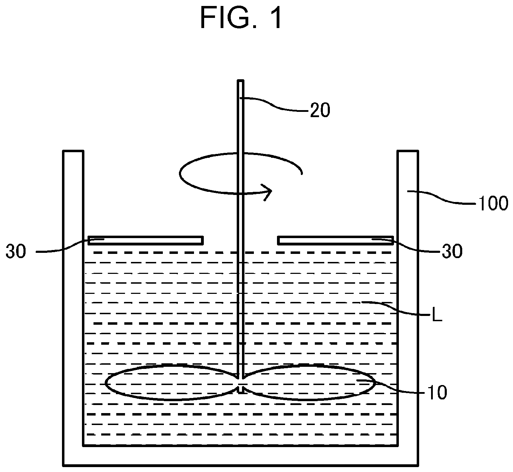

FIG. 1 is a schematic drawing showing the outlines of saccharification treatment according to an example. FIG. 1 shows a state of producing a cellulose-derived saccharide. A liquid L prepared by mixing a reaction solution, a raw material, and an enzyme is introduced into a saccharification tank 100, and an adsorption sheet 30 is disposed on the surface of the liquid L. The liquid L (the raw material, the reaction solution, the enzyme, etc.) is stirred by rotation of a stirring shaft 20 and a stirring blade 10 using a motor not shown.

Stirring causes the cellulose to be saccharified by enzyme reaction in the liquid L. Also, stirring causes the adsorption sheet 30 to collect impurities such as oil components, including lignin, pitch, and the like, brought from the raw material. Further, impurities such as oil components, including lignin, pitch, and the like, released from cellulose with proceeding of the enzyme reaction are adsorbed by the adsorption sheet 30. 7. 2. Example of shape of adsorption sheet

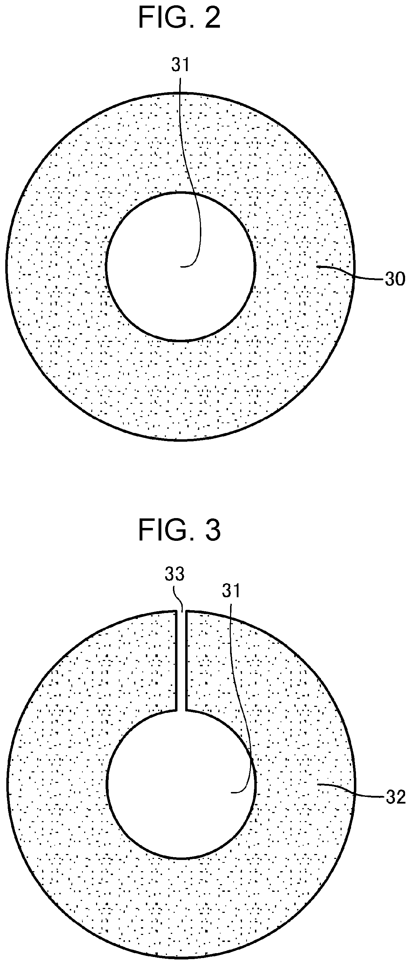

FIG. 2 is a schematic plan view of the adsorption sheet 30 according to the example. As shown in the drawing, the adsorption sheet 30 has, in a plan view, an annular shape having a circular outer shape and a hole 31 at a central portion. Although not shown in the drawing, the outer shape in a plan view is not limited to a circle and can be freely set according to the shape of the inner wall of the saccharification tank 100 or the like. Further, the hole 31 in a plan view is not limited to being formed at the central portion, and the hole 31 has any desired shape. Further, a plurality of holes 31 may be formed. The adsorption sheet 30 has the hole 31, and thus each of the main components can be supplied to the liquid L through the hole 31. Also, the hole 31 enables a stirring operation to be performed so as to avoid interference between the stirring shaft 20 and the adsorption sheet 30.

FIG. 3 is a schematic plan view of an adsorption sheet 32 according to a modified example. As shown in the drawing, the adsorption sheet 32 has, in a plan view, an annular shape having a circular outer shape, a hole 31 formed at a central portion, and a cut 33 formed to connect the outer periphery and the hole 31. In the adsorption sheet 32, the outer shape and the hole 31 are the same as in the adsorption sheet 30 described above. When the cut 33 is formed as in the adsorption sheet 32, the adsorption sheet 32 can be easily disposed on the liquid surface of the liquid L even after the stirring shaft 20 is installed at the predetermined position.

The inventors have obtained knowledge that impurities easily float near the inner wall during stirring. Therefore, as shown in FIG. 1 to FIG. 3, the adsorption sheet 30 or 32 is disposed near the inner wall of the saccharification tank 100, and thus impurities such as oil components, including lignin, pitch, and the like, are easily collected.

### 7. 3. Experimental example

The effect of the adsorption sheet on the efficiency of saccharification of cellulose was verified by experiment. Two sets were prepared by adding 50 g of used paper, 1 L of a sodium acetate buffer solution, and enzyme Cellic Ctec 2 (manufactured by Novozymes Inc.) to a 2 L beaker, and pH was adjusted to 5.2 by adding acetic acid. In one of the sets, a magnetic stirrer stir bar and an adsorption sheet (material: silicone rubber, thickness: 5 mm, shape: the shape cut out to match with the inner wall of the beaker) were placed. In the other set, only a magnetic stirrer sir bar was placed. In each of the two sets, the mixture was adjusted to a temperature of 49°C and reacted by stirring for 2 days.

Each of the liquids obtained after reaction was collected, and a difference in saccharide concentration due to the presence of the adsorption sheet was compared. The experimental method is as follows.
1. A plurality of reference saccharide solutions at known saccharide concentrations are prepared.
2. Then, 3 mL of a saccharide solution concentration measurement reagent (trade name "Glucose CII-Test Wako", manufactured by Fujifilm Wako Pure Chemical Corporation) is pipetted and stirred in each of the reference saccharide solutions prepared in (1) and 0.02 mL of a saccharification reaction solution diluted so that the saccharide solution concentration is 50 mg/dL or more and 500 mg/dL or less.
(3) Each of the liquids prepared in (2) is allowed to stand at 37°C for 5 minutes.
(4) The wavelength of each of the liquid colors is measured by absorbance measurement.
(5) A calibration curve for absorbance obtained at a wavelength of 505 nm is formed using the reference, and the saccharide solution concentration of the saccharification reaction solution is calculated.

As a result of the experiment, when the adsorption sheet was disposed, a saccharide concentration of 30 g/L was obtained, and the saccharide production rate (saccharification rate) was 60% relative to the total amount of cellulose. While when the adsorption sheet was not disposed, a saccharide concentration of 23 g/L was obtained, and the saccharide production rate (saccharification rate) was 45% relative to the total amount of cellulose. Thus, the significant effect of improving the saccharification rate by 15% was confirmed when the adsorption sheet was disposed.

The exemplary embodiment and the modified example are only examples and are not limited to these. For example, the exemplary embodiment and modified examples can be properly combined.

The present disclosure includes substantially the same configuration as that described in the embodiments, for example, a configuration having the same function, method, and results, or a configuration having the same object and effect. The present disclosure also includes a configuration in which a portion not essential in the configuration described in the embodiment is replaced. Further, the present disclosure includes a configuration which can exhibit the same operational effect or achieve the same object as in the configuration described in the embodiment. Further, the present disclosure includes a configuration in which a known technology is added to the configuration described in the embodiment.

The contents below are derived from the exemplary embodiment and the modified example.

A method for saccharifying cellulose includes introducing a cellulose-containing raw material and a reaction solution into a saccharification tank, introducing an enzyme into the reaction solution in the saccharification tank, disposing an adsorption sheet on the liquid surface of the reaction solution, and decomposing the cellulose with the enzyme by stirring the reaction solution to produce a saccharide, the adsorption sheet adsorbing an oil component released from the raw material to the liquid surface of the reaction solution during the production of the saccharide.

The method for saccharifying cellulose enables the adsorption sheet to collect impurities such as oil components, including lignin, pitch, and the like, in the raw material, and thus enzyme reaction is hardly inhibited, and the inside of the saccharification tank can be easily maintained clean. Therefore, cellulose can be efficiently saccharified, and thus a saccharide can be efficiently produced. Also, the inside of the saccharification tank is cleaner, and thus the saccharification tank after use can be easily washed.

In the method for saccharifying cellulose described above, the raw material may be paper.

In the method for saccharifying cellulose, the raw material can be easily procured.

In the method for saccharifying cellulose described above, the paper may contain printed used paper.

The method for saccharifying cellulose can efficiently produce a saccharide from used paper treated as a waste, and thus can preferably cope with saving of environmental resources, underground resources, or the like. Further, the printed used paper contains oil components, such as ink and the like, adhered thereto, but the oil components can be preferably removed by the adsorption sheet. Thus, the effect of improving the saccharification efficiency can be more remarkably obtained, and the effect of facilitating washing of the saccharification tank can also be more remarkably obtained.

In the method for saccharifying cellulose described above, the raw material may be introduced in a crushed state into the saccharification tank.

In the method for saccharifying cellulose, unnecessary components contained in the raw material can be easily released, and cellulose can be easily brought into contact with the reaction solution, thereby enabling to more improve the saccharification efficiency

In the method for saccharifying cellulose described above, the adsorption sheet may have a network structure.

In the method for saccharifying cellulose, the adsorption sheet easily collects oil components as impurities, such as lignin, pitch, and the like, in the raw material, and thus the saccharification efficiency can be further enhanced.

In the method for saccharifying cellulose described above, the adsorption sheet may have an annular shape in a plan view.

In the method for saccharifying cellulose, the shaft of a stirring blade can be passed through the central hole of the annular of the adsorption sheet, and an additional component can be added to the saccharification tank through the central hole, thereby enabling to further enhance the saccharification efficiency.

In the method for saccharifying cellulose described above, the adsorption sheet may be disposed near the inner wall of the saccharification tank.

In the method for saccharifying cellulose, the adsorption sheet is disposed near the inner wall of the saccharification tank, where impurities such as oil components, including lignin, pitch, and the like, in the raw material are easily collected, and thus the impurities can be more efficiently collected by the adsorption sheet.

## Claims

1. A method for saccharifying cellulose comprising:
introducing a cellulose-containing raw material and a reaction solution into a saccharification tank;
introducing an enzyme into the reaction solution in the saccharification tank;
disposing an adsorption sheet on the liquid surface of the reaction solution; and
decomposing the cellulose with the enzyme by stirring the reaction solution to produce a saccharide,
wherein the adsorption sheet adsorbs an oil component released from the raw material to the liquid surface of the reaction solution during the production of the saccharide.

2. The method for saccharifying cellulose according to claim 1, wherein the raw material is paper.

3. The method for saccharifying cellulose according to claim 2, wherein the paper contains printed used paper.

4. The method for saccharifying cellulose according to claim 1, wherein the raw material is introduced in a crushed state into the saccharification tank.

5. The method for saccharifying cellulose according to claim 1, wherein the adsorption sheet has a network structure.

6. The method for saccharifying cellulose according to claim 1, wherein the adsorption sheet has an annular shape in a plan view.

7. The method for saccharifying cellulose according to claim 1, wherein the adsorption sheet is disposed near the inner wall of the saccharification tank.
